# EUROPÄISCHE PATENTANMELDUNG

(11) **EP 0 547 354 A1**
(43) Veröffentlichungstag der Anmeldung: **23.06.1993**
(21) Anmeldenummer: 92118859.5
(22) Anmeldetag: 04.11.1992
(51) Int. Cl.: A61F 2/36

(54) **Einstellbare Hüftgelenk-Endoprothese**

(30) Priorität: 17.12.1991 DE 4141527
(71) Anmelder: Dr.Ing.h.c. F. Porsche Aktiengesellschaft, D-70435 Stuttgart (DE)
(72) Erfinder: Rispeter, Siegfried, W-7122 Besigheim (DE)

(57) **Zusammenfassung**

Für eine Hüftgelenk-Endoprothese mit einem einstellbaren Prothesenkopf, der mit einem im Oberschenkelknochen festsetzbaren Stiel verbunden ist, wird vorgeschlagen, Überlasten, die zu einer Beschädigung des in den Oberschenkelknochen implantierten Prothesenteiles und damit auch zu einer erneuten Implantation führen würden, in einem Begrenzungselement aufzufangen. Das Begrenzungselement ist im Prothesenkopf angeordnet und nimmt die Überlast durch Verformung, Bruch oder, im Falle einer reibschlüssigen Verbindung, durch Rutschen auf. Es muß nach dem Auftreten einer Überlast ersetzt bzw. neu eingestellt werden.

## Beschreibung

Die Erfindung betrifft eine Hüftgelenk-Endoprothese nach dem Oberbegriff des Anspruchs 1.

Die nicht vorveröffentlichte DE-A-41 27 981 offenbart eine Hüftgelenk-Endoprothese mit einem einstellbaren Prothesenkopf, der mit einem im Oberschenkelknochen festsetzbaren Stiel verbunden ist. Der Prothesenkopf ist mehrteilig und in drei Freiheitsgraden einstellbar ausgeführt. Mittels Verzahnungen wird für eine (fein)stufige Einstellbarkeit einer Verschiebung in medial-lateraler Richtung und einer Drehung um eine Achse senkrecht zu dieser Richtung gesorgt. Die Sicherung erfolgt über eine einzige Schraube, die die im Eingriff stehenden Verzahnungen gegeneinander verspannt hält. Eine Einstellung der Höhe der auf den Prothesenkopf über einen Konus aufgesetzten Gelenkkugel erfolgt durch Gelenkkugeln mit unterschiedlich tief eingepreßten Konen, die eine Verschiebung des Gelenkkugelmittelpunktes in proximal-distaler Richtung bewirken.

Aufgabe der Erfindung ist es, eine verbesserte einstellbare Hüftgelenk-Endoprothese zu schaffen, die im Überlastfall eine Beschädigung des im Oberschenkelknochen implantierten Teiles einer Küftgelenk-Endoprothese verhindert.

Erfindungsgemäß wird diese Aufgabe durch die Merkmale des Anspruchs 1 gelöst. Weitere, die Erfindung ausgestaltende Merkmale sind in den Unteransprüchen gekennzeichnet.
Mit der Erfindung wird in vorteilhafter Weise eine Hüftgelenk-Endoprothese geschaffen, die im Überlastfall eine Beschädigung des im Oberschenkelknochen implantierten Teiles der Hüftgelenk-Endoprothese und gleichzeitig auch eine damit einhergehende Beschädigung des Oberschenkelknochens verhindert.

Die Begrenzung der Kräfte erfolgt durch Deformation oder Bruch eines Materialquerschnittes oder Rutschen eines Reibschlußelementes im Prothesenkopf. Im Falle der Deformation und des Rutschens kann die Prothese noch für kurze Zeit weiterverwendet werden. In jedem Fall wird hierdurch eine mit großen Risiken verbundene Neuimplantation vermieden. Stattdessen muß nur der Teil des Prothesenkopfes ausgetauscht werden, der als Kraftbegrenzungseinrichtung den Kraftanteil durch Bruch oder Deformation aufgenommen hat, der einen festgelegten Höchstwert übersteigt. Ist die Kraftbegrenzungseinrichtung als Reibschlußelement ausgeführt, so ist nicht einmal ein Austausch notwendig, sondern nur eine Neueinstellung zur Wiederherstellung der Geometrie der Hüftgelenk-Endoprothese.

Durch Deformation oder Bruch eines Materialquerschnittes oder Rutschen eines Reibschlußelementes wird eine Überlastung durch eine extreme Fehlstellung des Oberschenkels deutlich sichtbar und spürbar gemacht.

Der Überlastfall ist durch die Überschreitung des Höchstwertes der von der Hüftgelenk-Endoprothese auf den Oberschenkelknochen übertragbaren Kraft charakterisiert. Der Höchstwert der von der Kraftbegrenzungseinrichtung übertragbaren Kraft ist so gewählt, daß der Überlastfall nicht erreicht wird, jedoch im Alltag auftretende Kräfte darunter liegen. Kleinere Überschreitungen des Höchstwertes, wie sie in Ausnahmesituationen auftreten können, führen bei den für die Prothesen üblicherweise verwendeten Materialien bei impulsartiger Wirkdauer nicht zu einem Ansprechen der Kraftbegrenzungseinrichtung, sondern werden übertragen. Überschreitungen im Bereich des Überlastfalles hingegen müssen zum sofortigen Ansprechen der Kraftbegrenzungseinrichtung führen.

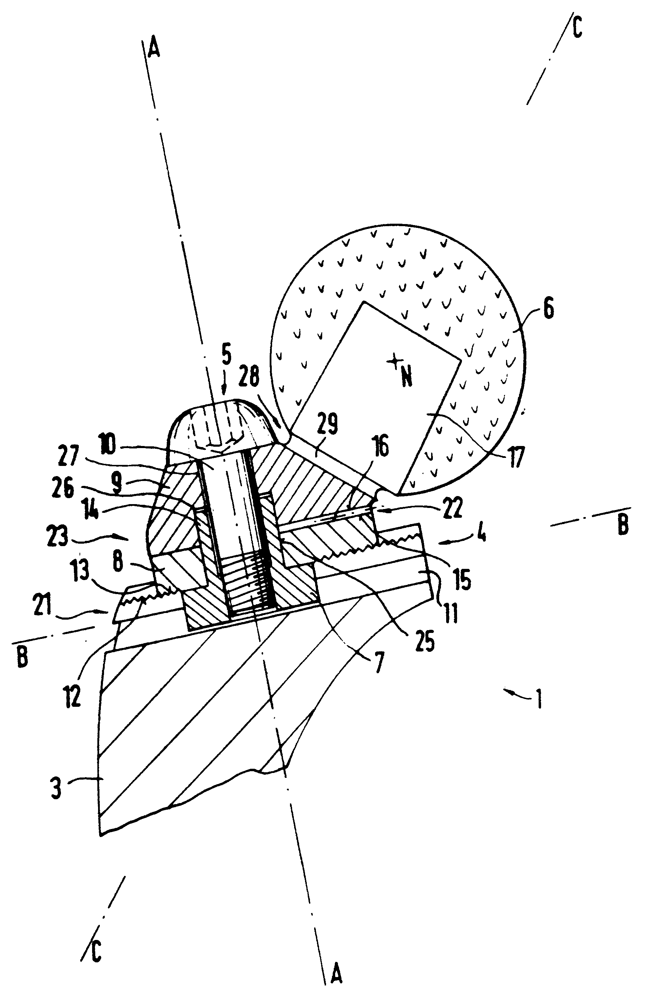

Ein Ausführungsbeispiel der Erfindung ist in der Zeichnung dargestellt und wird nachfolgend näher beschrieben.

Die einzige Figur zeigt einen Schnitt durch einen oberen Bereich 1 einer einstellbaren Hüftgelenk-Endoprothese mit einer Einstelleinrichtung 23 in einer Frontalebene. Die Hüftgelenk-Endoprothese besteht aus einem Stiel 3, einem hierin eingearbeiteten Kulissenbereich 4 sowie einem gegenüber dem Kulissenbereich 4 beweglichen Prothesenkopf 5, welcher eine Gelenkkugel 6 trägt und der ein Kulissenelement 7, eine Zwischenplatte 8 und einen Tragkörper 9 mit einem Spannelement, das aus einem Gewindebolzen 10 bestehen kann, umfaßt. Zwischen dem Tragkörper 9 und der Zwischenplatte 8 einerseits und dem Kulissenbereich 4 sowie der Zwischenplatte 8 andererseits sind aus Stirnverzahnungen 12, 13 und 15, 16 gebildete Arretiermittel 21, 22 angeordnet. Die Hüftgelenk-Endoprothese ist in einen Oberschenkelknochen eingesetzt und dort fixiert. Die Gelenkkugel 6 steht in einer mit N bezeichneten Ausgangsstellung.

Zur Einstellung einer orthopädisch korrekten Lage der Gelenkkugel 6 sind mindestens drei Einstellrichtungen vorgesehen. Eine erste Einstellung erfolgt durch lineare Verschiebung in medial-lateraler Richtung nach einer Linie B-B, eine zweite Einstellung erfolgt in proximal-distaler Richtung nach einer Linie C-C und eine dritte Einstellung erfolgt durch Drehung um eine Achse A-A, die gesehen auf die Frontalebene senkrecht auf der Linie B-B steht.

Der Kulissenbereich 4 weist an seiner dem Prothesenkopf 5 zugewandten Oberfläche eine hinterschnittene Nut 11 in medial-lateraler Richtung auf, in die das Kulissenelement 7 eingreift. Durch die Hinterschneidung kann das Kulissenelement 7 aus dem Gewindebolzen herrührende Spannkräfte senkrecht zur Richtung der Nut 11 übertragen. Auf der Oberfläche des Kulissenbereichs 4 ist die erste lineare Stirnverzahnung 12 angeordnet. Oberhalb des Kulissenbereichs 4 lagert die Zwischenplatte 8, auf deren dem Kulissenbereich 4 zugewandter Oberfläche die zweiten lineare Stirnverzahnung 13 angeordnet ist, die das Gegenstück zur ersten linearen Stirnverzahnung 12 bildet und in diese eingreift. Die Stirnverzahnung 12 erstreckt sich über die gesamte Länge des Kulissenbereichs 4 und ist nur auf einer Seite angeordnet. Auf der gegenüberliegenden Seite ist eine nicht dargestellte prismatischen Führung vorgesehen, die den Prothesenkopf 5 gegenüber dem Stiel 3 führt.

Eine Bohrung 25 in der Zwischenplatte 8 nimmt einen Zapfen 14 des Kulissenelementes 7 auf. Radial zum Mittelpunkt dieser Bohrung 25 und gegenüberliegend der zweiten linearen Stirnverzahnung 13 auf der Oberseite der Zwischenplatte 8, ist die erste radiale Stirnverzahnung 15 des Arretiermittels 22 erhaben angeordnet, die von einer etwa kreissegmentförmigen Fläche begrenzt ist.

Der Tragkörper 9 liegt auf der Zwischenplatte 8 auf, wobei der Zapfen 14 des Kulissenelementes 7 in eine Sackbohrung 26 des Tragkörpers 9 ragt. In Verlängerung dieser Bohrung 26 ist eine im Durchmesser kleinere Bohrung 27 vorgesehen, durch die der Gewindebolzen 10 durch den Tragkörper 9 hindurch in ein Innengewinde im Zapfen 14 des Kulissenelementes 7 eingeschraubt wird. Gegenüberliegend und korrespondierend zur ersten radialen Stirnverzahnung 15 trägt der Tragkörper 9 eine zweite radiale Stirnverzahnung 16. Eine diese Stirnverzahnung 16 begrenzende kreissegmentförmige Fläche umfaßt einen kleineren Winkel als die die erste radiale Stirnverzahnung 15 begrenzende Fläche.

Der Tragkörper 9 ist mit einem Schaft versehen, der an seinem Ende einen Außenkonus 17 trägt. Auf diesen ist die Gelenkkugel 6 aufgesetzt, in die ein gleichartiger Innenkonus eingearbeitet ist. Zwischen dem Schaft und dem Außenkonus 17 ist als Kraftbegrenzungseinrichtung 28 eine umlaufende Kerbe 29 vorgesehen, die nach Form und Tiefe so gestaltet ist, daß in dem von der Kerbe 29 begrenzten Materialquerschnitt die Materialfestigkeit etwas früher erschöpft ist als in allen anderen im Kraftfluß von der Gelenkkugel 6 zum Stiel 3 liegenden Materialquerschnitten, d.h. eine Sollbruchstelle geschaffen wird.

Die Materialfestigkeit wird vorzugsweise so gewählt, daß alle alltäglichen Belastungen sicher und dauerfest übertragen werden. Kurze Belastungen oberhalb der Materialfestigkeit führen bei den für Prothesen in der Regel verwendeten Stählen nicht sofort zum Bruch, werden also noch übertragen. Erst längerdauernde Belastungen oder Belastungen deutlich oberhalb dieser Größenordnung führen zum sofortigen Bruch in dem von der Kerbe 28 begrenzten Materialquerschnitt.

Nach dem Bruch ist eine Kraftübertragung über die Hüftgelenk-Endoprothese nicht mehr möglich. Es muß jedoch nur der beschädigte Tragkörper 9 in einer Operation ausgetauscht werden, während insbesondere der im Oberschenkelknochen verankerte Stiel 3 und der Oberschenkelknochen selbst nicht in Mitleidenschaft gezogen sind.

In weiterer, nicht dargestellter Ausführung der Erfindung ist es vorteilhafterweise vorgesehen, daß Überlastungen nicht zu einem Bruch, sondern zu Verformungen führen. Dies kann beispielsweise durch eine Gestaltung des Schaftes des Tragkörpers 9 im Falle einer Druckbelastung als Hohlprofil oder im Falle einer Biegebelastung als Biegebalken erfolgen. Nach einer Überlastung ist eine Kraftübertragung über die Hüftgelenk-Endoprothese weiterhin möglich, wenn auch die durch die Verformung eingetretenen Veränderungen einen baldigen Austausch des Tragkörpers 9 notwendig machen.

Die Kraftbegrenzungseinrichtung 28 kann z.B. auch als Reibschlußelement ausgeführt sein. Diese nimmt Kraftspitzen, die die Reibkraft übersteigen, durch Rutschen auf. Hierdurch wird die Prothesengeometrie verändert, die dann durch eine erneute Einstellung wiederhergestellt werden muß.

## Patentansprüche

1. Hüftgelenk-Endoprothese mit einem einstellbaren Prothesenkopf (5), der mit einem im Oberschenkelknochen festsetzbaren Stiel (3) verbunden ist, **dadurch gekennzeichnet**, daß der Prothesenkopf (5) eine über Arretiermittel (21,22) fixierbare Einstelleinrichtung (23) mit einem in einem ortsfesten Kulissenbereich geführten Kulissenelement (7) umfaßt, auf dem ein Tragkörper (9) einer Gelenkkugel (6) gehalten und über eine Zwischenplatte (8) zum Kulissenbereich (4) verstellbar abgestützt ist, und die Arretiermittel (21,22) über ein die Einrichtung (23) durchdringendes Spannelement gegeneinander festsetzbar sind, wobei im Prothesenkopf (5) eine Kraftbegrenzungseinrichtung (28) derart vorgesehen ist, daß eine von der Hüftgelenk-Endoprothese zu übertragende Kraft auf einen Höchstwert begrenzbar ist.

2. Hüftgelenk-Endoprothese nach Anspruch 1, **dadurch gekennzeichnet**, daß die Kraftbegrenzungseinrichtung (28) einen verminderten Materialquerschnitt des Prothesenkopfes (5) umfasst, der bei Erreichen des Krafthöchstwertes eine Sollbruchstelle bildet.

3. Hüftgelenk-Endoprothese nach Anspruch 1, **dadurch gekennzeichnet**, daß die Kraftbegrenzungseinrichtung (28) einen verminderten Materialquerschnitt des Prothesenkopfes (5) umfasst, der bei Erreichen des Krafthöchstwertes eine Solldeformationsstelle bildet.

4. Hüftgelenk-Endoprothese nach Anspruch 1, 2 oder 3, **dadurch gekennzeichnet**, daß die Kraftbegrenzungseinrichtung (28) zwischen dem Prothesenkopf (5) und einem Konus (17) vorgesehen ist und aus einer Kerbe (29) besteht.

5. Hüftgelenk-Endoprothese mit einem einstellbaren Prothesenkopf (5), der mit einem im Oberschenkelknochen festsetzbaren Stiel (3) verbunden ist, **gekennzeichnet durch** folgende Merkmale:
a. ein Kulissenbereich (4) weist eine in Richtung einer medial-lateralen Verschiebung (B-B) verlaufende hinterschnittenen Nut (11) auf und ist an einer Oberfläche und senkrecht zu dieser Nut (11) mit einer ersten linearen Stirnverzahnung (12) versehen;
b. ein Kulissenelement (7) ist in der Nut (11) geführt, dessen zylindrischer Zapfen (14) eine Bohrung mit einem Innengewinde aufweist;
c. eine Zwischenplatte (8) liegt auf dem Kulissenbereich (4) auf, wobei eine Bohrung (25) den Zapfen (14) aufnimmt und eine zweite lineare Stirnverzahnung (13) an der Zwischenplatte (8) in Eingriff mit der ersten Stirnverzahnung (12) des Kulissenbereiches (4) steht;
d. gegenüberliegend der zweiten Stirnverzahnung (13) ist an der Oberseite der Platte (8) eine erste radiale Stirnverzahnung (15) radial zum Mittelpunkt der Bohrung (25) vorgesehen und von einer kreissegmentförmigen Fläche begrenzt;
e. ein Tragkörper (9) liegt auf der Zwischenplatte (8) auf, wobei eine Sackbohrung (26) den zylindrischen Zapfen (14) des Kulissenelements (7) aufnimmt und eine zweite radiale Stirnverzahnung (16) am Tragkörper (9) so angeordnet ist, daß sie in Eingriff mit der ersten radialen Stirnverzahnung (15) der Platte (8) steht und ein schräg-radial zur Bohrung ausladender Schaft einen Konus (17) zur Aufnahme einer Gelenkkugel (6) trägt;
f. die Gelenkkugel (6) ist mit einer kegeligen Ausnehmung entsprechend dem Konus versehen, wobei die Einpreßtiefe dieser kegeligen Ausnehmung durch herstellungsseitige Maßnahmen eingestellt ist,
g. ein einziger Gewindebolzen (10) ist durch eine annähernd senkrecht zur Richtung der medial-lateralen Verschiebung (B-B) verlaufenden Bohrung (27) im Tragkörper (9) mit dem Innengewinde des Zapfens (14) in Verbindung und hält im angezogenen Zustand Kulissenbereich (4), Zwischenplatte (8) und Tragkörper (9) und somit auch die im Eingriff stehenden Arretiermittel (21,22) gegeneinander verspannt und
h.der Tragkörper (9) ist an dem schräg-radial ausladenden Schaft am Ansatz des Konus (17) mit einer aus einer umlaufenden Kerbe (29) bestehenden Kraftbegrenzungseinrichtung (28) versehen, die derart ausgeführt ist, daß der Schaft bei Erreichen einer unter den Höchstkräften aller anderen Elemente liegenden Höchstkraft vom Konus (17) trennbar ist.
